# EUROPEAN PATENT APPLICATION

(11) **EP 4 834 339 A1**
(43) Date of publication of application: **04.11.2026**
(21) Application number: 24911303.6
(22) Date of filing: 26.12.2024
(51) Int. Cl.: A61K 31/192, A61K 31/136, A61K 31/145, A61K 9/107, A61K 9/06, A61K 9/00, A61P 17/10

(54) **COMPOUND COMPOSITION OF ADAPALENE AND DAPSONE, PREPARATION METHOD THEREFOR, AND USE THEREOF**

(30) Priority: 28.12.2023 CN 202311832033
(71) Applicant: Hangzhou Solipharma Co., Ltd., Hangzhou, Zhejiang 310018 (CN)
(72) Inventor: SHENG, Xiaoxia, Hangzhou, Zhejiang 310018 (CN); YAN, Bo, Hangzhou, Zhejiang 310018 (CN)
(74) Representative: Müller-Boré & Partner Patentanwälte PartG mbB
(86) International application number: PCT/CN2024/142652
(87) International publication number: WO 2025/140381

(57) **Abstract**

The present disclosure belongs to the field of pharmaceutical formulations, and particularly relates to a composition comprising adapalene and dapsone for topical dermal use, a preparation method and use thereof. The composition of the present disclosure has at least one of the following excellent characteristics: high solubility of the active ingredients in a carrier, small particle size of drug particles, stable particle size, good skin absorption, high in-vitro release efficiency, small oil particle size of composition, good stability, good therapeutic effect, low skin irritation, and good storage stability. The composition of the present disclosure may be directly used as a formulation or may be further formulated into different dosage forms after addition of other carriers.

## Description

### Cross-Reference to Related Applications

The present application claims all the benefits of the Chinese patent application No. 202311832033.8 filed on December 28, 2023 with the National Intellectual Property Administration of the People's Republic of China, which is incorporated by reference in its entirety into the present application.

### Field

The invention relates to the technical field of pharmaceutical preparations. Specifically, the present invention relates to a composition comprising adapalene and dapsone for topical skin application, a preparation method thereof, and uses thereof.

### Background

Adapalene and dapsone are commonly used drugs for treating acne. Currently, the marketed products of adapalene include 0.1% and 0.3% gels, as well as 0.1% cream, and the marketed products of dapsone include 5% and 7.5% gels.

Adapalene is poorly soluble in both water and oil. Due to its strong hydrophobicity, adapalene has low solubility in the matrix and is often dispersed in a crystalline state within the gel matrix, resulting in poor drug absorption and bioavailability. Previous studies have shown that adapalene has a very low absorption rate, whether in the form of gel or cream, and is prone to skin allergies and irritation. Furthermore, adapalene creams also have the problem of poor skin comfort when applied.

The marketed dapsone gel suffers from storage instability and large particle size. After long-term storage, the dapsone particles in the gel gradually increase in size, affecting product stability and causing a gritty feeling during use. Furthermore, particle size instability in topical preparations can lead to significant variations in release and absorption rates during use, impairing drug efficacy.

Therefore, existing topical acne treatment products, such as dapsone and adapalene, each have their own challenges, such as low drug solubility in the carrier, large particle size, particle size growth during storage, poor stability, and poor skin absorption. There is a need to develop a topical product that addresses these issues and achieves improved stability, compliance, and bioavailability.

### Summary

To produce a topical acne product with excellent drug solubility, a suitable and stable particle size, and good bioavailability, the present invention provides a composition comprising adapalene and dapsone. This composition exhibits at least one of the following advantageous properties: high solubility of the active pharmaceutical ingredient in the carrier, small and stable drug particle size, good skin absorption, high in vitro release efficiency, small oil droplet size, good stability, good therapeutic effect, low skin irritation, and good shelf stability. The invention composition can be used directly as a preparation or further prepared into various dosage forms by adding other carriers.

In one aspect of the present invention, a composition comprising adapalene and dapsone is provided. Based on the weight of the total composition, the composition comprises 1%-15% of dapsone, 0.05%-0.5% of adapalene, 0.25%-20% of a carboxylic acid ester-based oily solvent, 0.5%-20% of a polyoxyethylene nonionic surfactant, 5%-50% of diethylkene glycol monoethyl ether, and other pharmaceutically acceptable carriers.

In the preferred technical solution of the present invention, based on the total weight of the composition, the composition comprises 0.25-15% of a carboxylic acid ester-based oily solvent.

In some embodiments, based on the total weight of the composition, the composition comprises 0.25%-10%, 0.25%-5%, 0.25%-4%, 0.25%-3%, 0.25%-2%, 0.25%-1%, 0.5%-10%, 0.5%-5%, 0.5%-1%, 1%-10%, 1%-5%, 5%-10% or 10%-15% of a carboxylic acid ester-based oily solvent.

In some embodiments, based on the total weight of the composition, the composition comprises 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15% of a carboxylic acid ester-based oily solvent.

In the preferred technical solution of the present invention, the carboxylate ester-based oily solvent is selected from any one of monocarboxylic acid esters, dicarboxylic acid esters, and tricarboxylic acid esters, or a mixture thereof; preferably, the oily solvent is a monocarboxylic acid ester.

In some embodiments, the monocarboxylic acid ester is selected from any one of propylene glycol monocaprylate, propylene glycol monolaurate, propylene glycol oleate, propylene glycol myristate, isopropyl palmitate, lauryl lactate, isopropyl myristate, ethyl oleate, ethyl linoleate, glyceryl monolinoleate, glyceryl monostearate, and glyceryl monooleate, or a mixture thereof; the dicarboxylic acid ester is selected from any one of propylene glycol dicaprylate, diisopropyl adipate, diethyl sebacate, propylene glycol dicaprate, and dinbutyl adipate, or a mixture thereof; the tricarboxylic acid esters is selected from any one of medium-chain triglycerides, glyceryl triacetate, and caprylic capric triglyceride, or a mixture thereof;

Furthermore, the carboxylic acid ester-based oily solvent is selected from any one of propylene glycol monocaprylate and propylene glycol monolaurate, or a mixture thereof.

In some embodiments, the carboxylic acid ester-based oily solvent is propylene glycol monocaprylate.

In some embodiments, the propylene glycol monocaprylate is a commercially available product Capryol.

In some embodiments, the propylene glycol monocaprylate is commercially available product Capryol 90, a mixture of propylene glycol monoester and propylene glycol diester, having a monoester content > 90% and a diester content <10%.

In some embodiments, the carboxylic acid ester-based oily solvent is propylene glycol monolaurate.

In some embodiments, the propylene glycol monolaurate is a commercially available product from the Lauroglycol series.

In some embodiments, the propylene glycol monolaurate is commercially available product Lauroglycol 90, a mixture of propylene glycol monoester and propylene glycol diester, having a monoester content >90% and a diester content <10%.

In the preferred technical solution of the present invention, based on the total weight of the composition, the composition comprises 0.5-15% of a polyoxyethylene nonionic surfactant.

In some embodiments, based on the total weight of the composition, the composition comprises 0.5-10%, 0.5%-5%, 0.5%-4%, 0.5%-3%, 0.5%-2%, 0.5%-1%, 1%-10%, 1%-5%, 5%-10% or 10%-15% of a polyoxyethylene nonionic surfactant.

In some embodiments, based on the total weight of the composition, the composition comprises 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14.%, 14.5%, and 15% of a polyoxyethylene nonionic surfactant.

In the preferred technical solution of the present disclosure, the polyoxyethylene nonionic surfactant is selected from any one of polyoxyethylene castor oil, fatty alcohol polyoxyethylene ether, polyol polyoxyethylene ether fatty acid ester, alkylphenol polyoxyethylene ether, fatty acid polyoxyethylene ester and fatty amine polyoxyethylene ether, or a mixture thereof.

Furthermore, the polyoxyethylene nonionic surfactant is polyoxyethylene castor oil.

In some embodiments, the polyoxyethylene castor oil is selected from any one of polyoxyethylene 35 castor oil, polyoxyethylene 40 hydrogenated castor oil, polyoxyethylene 54 hydrogenated castor oil and polyoxyethylene 60 hydrogenated castor oil, or a mixture thereof.

Furthermore, the polyoxyethylene castor oil is selected from any one of polyoxyethylene 35 castor oil and polyoxyethylene 40 hydrogenated castor oil or a mixture thereof.

In some embodiments, the polyoxyethylene nonionic surfactant is polyoxyethylene 35 castor oil.

In some embodiments, the polyoxyethylene nonionic surfactant is polyoxyethylene 40 hydrogenated castor oil.

In the preferred technical solution of the present invention, based on the total weight of the composition, the composition comprises 10%-50% of diethylene glycol monoethyl ether, more preferably 15%-45%.

In some embodiments, based on the total weight of the composition, the composition comprises 20%-45%, 20%-40%, 20%-35%, 20%-30%, 25%-45%, 25%-40%, 25%-35%, 30%-45%, 30%-40%, 35%-45%, or 40%-45% of diethylene glycol monoethyl ether.

In some embodiments, based on the total weight of the composition, the composition comprises 10%, 15%, 20%, 25%, 30%, 35%, 40% or 45% diethylene glycol monoethyl ether.

In the preferred technical solution of the present invention, based on the total weight of the composition, the composition comprises 2%-12% of dapsone.

In some embodiments, based on the total weight of the composition, the composition comprises 2%-11%, 2%-10%, 2%-9%, 2%-8%, 2%-7%, 2%-6%, 2%-5%, 2%-4%, 2%-3%, 3%-11%, 3%-10%, 3%-9%, 3%-8%, 3%-7%, 3%-6%, 3%-5%, 3%-4%, 4%-11%, 4%-10%, 4%-9.0%, 4%-8%, 4%-7%, 4%-6%, 5%-11%, 5%-10%, 5%-9%, 5%-8%, 5%-7%, 5%-6%, 6%-11%, 6%-10%, 6%-9%, 6%-8%, 6%-7%, 7%-11%, 7%-10%, 7%-9%, 7%-8%, 8%-11%, 8%-10%, 8.0%-9%, 9%-11%, 9%-10%, or 10%-11% dapsone.

In some embodiments, based on the total weight of the composition, the composition comprises 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5% or 12% dapsone.

In some embodiments, based on the total weight of the composition, the composition comprises 0.05%-0.4%, 0.05%-0.3%, 0.05%-0.2%, 0.05%-0.1%, 0.1%-0.4%, 0.1%-0.3%, 0.1%-0.2%, 0.2%-0.4%, 0.2%-0.3%, or 0.3%-0.4% adapalene.

In some embodiments, based on the total weight of the composition, the composition comprises 0.05%, 0.075%, 0.1%, 0.15%, 0.2%, 0.25%, 0.3%, 0.35%, 0.4%, 0.45% or 0.5% adapalene.

In some embodiments, based on the total weight of the composition, the amounts of dapsone and adapalene in the composition are 7.5% and 0.1%, 6.0% and 0.1%, 5.0% and 0.1%, 7.5% and 0.15%, 6.0% and 0.15%, 5.0% and 0.15%, 7.5% and 2.0%, 6.0% and 2.0%, 5.0% and 2.0%, 7.5% and 3.0%, 6.0% and 3.0%, 5.0% and 3.0%, 12% and 0.3%, or 3% and 0.075%, respectively.

In a preferred technical solution of the present disclose, the weight ratio of the polyoxyethylene nonionic surfactant to the carboxylic acid ester-based oily solvent in the composition is ≥ 1:1 or >1:1.

Furthermore, the weight ratio of the polyoxyethylene nonionic surfactant to the carboxylic acid ester-based oily solvent is ≤5:1, for example ≤4:1 or ≤3:1.

In some embodiments, the weight ratio of the polyoxyethylene nonionic surfactant to the carboxylic acid ester-based oily solvent is 1.5:1, 2:1, 2.5:1, 3:1, 3.5:1, 4:1 or 4.5:1.

In the preferred technical solution of the present disclose, the other pharmaceutically acceptable carriers include a crystallization inhibitor, and based on the total weight of the composition, the composition comprises 0.5%-4% of the crystallization inhibitor.

In some embodiments, based on the total weight of the composition, the composition comprises 0.5%-3.5%, 0.5%-3%, 0.5%-2.5%, 0.5%-2%, 0.5%-1.5%, 1%-3.5%, 1%-3%, 1%-2.5%, 1%-2%, 1.5%-3.5%, 1.5%-3%, or 1.5%-2.5% of the crystallization inhibitor.

In some embodiments, based on the total weight of the composition, the composition comprises 0.5%, 1%, 1.5%, 2%, 2.5%, 3%, 3.5%, or 4% of the crystallization inhibitor.

Furthermore, the crystallization inhibitor is selected from any one of povidone (PVP), polyvinyl alcohol (PVA) and hydroxypropyl cellulose (HPC) or a combination thereof.

In some embodiments, the crystallization inhibitor is PVP.

Furthermore, the polyvidone (PVP) is selected from any one of PVP-K12, PVP-K17, PVP-K25, PVP-K30, PVP-K60, and PVP-K90, or a combination thereof.

In some embodiments, the crystallization inhibitor is PVP-K30.

In a preferred technical solution of the present disclose, the pharmaceutically acceptable carrier further comprises a polymeric emulsifier. Based on the total weight of the composition, the composition comprises 0.05%-6% of the polymeric emulsifier.

Furthermore, based on the total weight of the composition, the composition comprises 0.05%-1% of the polymeric emulsifier.

In some embodiments, based on the total weight of the composition, the composition comprises 0.05%-0.9%, 0.05%-0.8%, 0.05%-0.7%, 0.05%-0.6%, 0.05%-0.5%, 0.05%-0.4%, 0.05%-0.3%, 0.05%-0.2%, 0.05%-0.1%, 0.1%-0.9%, 0.1%-0.8%, 0.1%-0.7%, 0.1%-0.6%, 0.1%-0.5%, 0.1%-0.4%, 0.1%-0.3%, 0.1%-0.2%, 0.2%-0.9%, 0.2%-0.8%, 0.2%-0.7%, 0.2%-0.6%, 0.2%-0.5%, 0.2%-0.4%, 0.2%-0.3%, 0.3%-0.9%, 0.3%-0.8%, 0.3%-0.7%, 0.3%-0.6%, 0.3%-0.5%, 0.3%-0.4%, 0.4%-0.9%, 0.4%-0.8%, 0.4%-0.7%, 0.4%-0.6%, 0.4%-0.5%, 0.5%-0.9%, 0.5%-0.8%, 0.5%-0.7%, 0.5%-0.6%, 0.6%-0.9%, 0.6%-0.8%, 0.6%-0.7%, 0.7%-0.9%, 0.7%-0.8% or 0.8%-0.9% of the polymeric emulsifier.

Furthermore, based on the total weight of the composition, the composition comprises 0.05%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95% or 1% of the polymeric emulsifier.

Furthermore, the polymeric emulsifier is a carbomer copolymer.

Still further, the carbomer copolymer is an acrylates/C10-30 alkyl acrylate crosspolymer.

In some embodiments, the acrylates/C10-30 alkyl acrylate crosspolymer is selected from commercially available products Pemulen TR-1, Pemulen TR-2, and Pemulen EZ-4U, or a combination thereof.

In some embodiments, the polymeric emulsifier is Pemulen TR-1.

In the preferred technical solution of the present disclose, the pharmaceutically acceptable carrier further comprises a thickening agent, and based on the total weight of the composition, the composition comprises 0.1% to 4% of the thickening agent.

Furthermore, the thickening agent is selected from polyacrylamide and carbomer homopolymer, or a combination thereof.

In some embodiments, the polyacrylamide comprises SEPINEO P 600, which is a mixture of acrylamide/sodium acryloyldimethyl taurate copolymer, isohexadecane, and polysorbate 80.

In some embodiments, the carbomer homopolymer is Carbomer 981.

Furthermore, the thickening agent is selected from SEPINEO P 600 and Carbomer 981, or a combination thereof.

In some embodiments, the thickening agent is a combination of SEPINEO P 600 and Carbomer 981.

In some embodiments, based on the total weight of the composition, the composition comprises 1%-3% SEPINEO P 600 and 0.1%-1% Carbomer 981.

In some embodiments, based on the total weight of the composition, the composition comprises 1%, 1.5%, 2%, 2.5%, or 3% SEPINEO P 600.

In some embodiments, based on the total weight of the composition, the composition comprises 0.1%, 0.2%, 0.3%, 0.4%, 0.5%, 0.6%, 0.7%, 0.8%, 0.9% or 1% Carbomer 981.

In some embodiments, based on the total weight of the composition, the composition comprises 1.5% SEPINEO P 600 and 0.2% Carbomer 981.

In the preferred technical solution of the present disclosure, the solubility of adapalene in the composition is at least 10 times higher than its solubility in water at the same temperature, for example, more than 100 times higher.

In the preferred technical solution of the present disclosure, the oil droplet diameter of the composition is ≤300 nm; for example, ≤250 nm.

In some embodiments, the composition has an oil droplet size of ≤ 240 nm, such as no more than 220 nm, no more than 200 nm, no more than 180 nm, no more than 160 nm, no more than 140 nm, 120 nm, no more than 100 nm, no more than 80 nm, no more than 60 nm, no more than 50 nm, no more than 40 nm, or no more than 30 nm.

In the preferred technical solution of the present disclosure, the transparency of the composition is not less than 50%, for example, not less than 80%.

In a preferred technical solution of the present disclosure, when the content of adapalene is 0.10% of the total weight of the composition, at least 10% of the adapalene is in a dissolved state.

In a preferred technical solution of the present disclosure, when the content of dapsone is 7.5% by weight of the total weight of the pharmaceutical composition of the topical dermatological composition, at least 50% of the dapsone is in a dissolved state.

In the preferred technical solution of the present disclosure, the composition may be directly applied to the skin or may be formulated with other pharmaceutically acceptable carriers to prepare other local pharmaceutical dosage forms.

In the preferred technical solution of the present disclosure, the composition is an emulsion or emulgel.

In some embodiments, the composition is an emulsion, such as a microemulsion.

In some embodiments, the composition is an emulsion, such as a microemulsion gel.

In the preferred technical solution of the present disclosure, the pharmaceutically acceptable carrier may further comprise any one selected ftom preservatives, antioxidants, chelating agents and a pH regulators, or a combination thereof.

In the technical solution provided by the present disclosure, the stability of the composition or the topical pharmaceutical formulation prepared therefrom may be further improved by the addition of preservatives, antioxidants, chelating agents and pH regulators, wherein the amounts of the additives are within commonly used concentration ranges for pharmaceutical excipients.

In some embodiments, the preservative is selected from methyl paraben (methylparaben), benzyl alcohol, sodium benzoate, ethyl paraben, propyl paraben, potassium sorbate, and phenoxyethanol, or a combination thereof.

In some embodiments, the preservative is methyl paraben (methylparaben).

In some embodiments, the antioxidant comprises any one selected from butylated hydroxyanisole (BHA), butylated hydroxytoluene (BHT), propyl gallate, and α-tocopherol, or a combination thereof.

In some embodiments, the antioxidant is butylated hydroxytoluene (BHT).

In the preferred technical solution of the present disclosure, the chelating agent is selected from any one ethylenediaminetetraacetic acid (EDTA), diethylenetriaminepentaacetic acid (DTPA), ethylenediamine-bis(o-hydroxyphenyl)acetic acid (EDDHA), hydroxy-2-ethylenediaminetriacetic acid (HEDTA), ethylenediamine-bis(o-hydroxy-p-methylphenyl)acetic acid (EDDHMA) and ethylenediamine-bis(5-carboxy-2-hydroxyphenyl)acetic acid (EDDCHA), or a combination of.

In some embodiments, the chelating agent is disodium edetate.

In the preferred technical solution of the present disclosure, the pH regulator comprises any one selected from sodium hydroxide and triethanolamine, or a combination thereof.

In some embodiments, the pH adjuster is sodium hydroxide.

In the preferred technical solution of the present disclosure, the pH of the composition is in the range of 5.0-7.0.

In some embodiments, the pH of the composition is in the range of 5.0-6.5.

In some embodiments, the pH of the composition is in the range of 5.0-6.0.

Another aspect of the present disclosure is to provide a method for preparing a composition comprising adapalene and dapsone, the preparation method comprising the following steps:
1) preparing an oil phase: adding dapsone and adapalene into a mixed solution of a carboxylic acid ester-based oily solvent, a polyoxyethylene nonionic surfactant, and diethylene glycol monoethyl ether to form an oil phase;
2) preparing an aqueous phase: uniformly dispersing other pharmaceutically acceptable carriers in water followed by mixing to form an aqueous phase; or directly using water as the aqueous phase;
3) emulsification: adding the oil phase prepared in step 1) to the aqueous phase prepared in step 2), adjusting the pH to 5.0-6.0, mixing uniformly, and adding water to make up to 100%.

In a preferred technical solution of the present disclosure, in step 2), the other pharmaceutically acceptable carriers are selected from any one of a polymeric emulsifier, a thickening agent and a crystallization inhibitor, or a combination thereof.

Furthermore, in step 2), the other pharmaceutically acceptable carriers are a polymer emulsifier, a thickening agent and a crystallization inhibitor.

Furthermore, in step 2), a thickening agent may be added after the polymeric copolymer emulsifier has been added.

Furthermore, in step 2), a crystallization inhibitor may be added prior to the addition of the polymeric copolymer emulsifier.

According to the preferred technical solution of the present disclosure, in steps 1) and/or 2), any one or a combination thereof selected from a penetration enhancer, an antioxidant, a preservative and a chelating agent may be further added.

Another aspect of the present disclosure provides a use of a composition comprising adapalene and dapsone for the preparation of a medicament for treating skin diseases.

According to an embodiment of the present disclosure, the skin disease is acne vulgaris, rosacea, rosacea, chronic wounds, bedsores, keratosis, sebaceous cysts, post-inflammatory hyperpigmentation, eczema, xerosis, pruritus, lichen planus, dermatitis, atopic dermatitis, prurigo nodularis, or prickly heat.

Furthermore, the skin disease is acne vulgaris.

According to an embodiment of the present disclosure, the acne vulgaris is mild, moderate, moderately severe or severe acne;

Preferably, the acne vulgaris is moderate or moderately severe acne.

Another aspect of the present disclosure provides a use of a composition comprising adapalene and dapsone for the preparation of a topically administered pharmaceutical formulation.

According to an embodiment of the present disclosure, the topically administered pharmaceutical formulation is selected from emulsions, emulgels, and creams.

Another aspect of the present disclosure provides a method for treating skin diseases, comprising administering to a subject in need thereof a therapeutically effective amount of the composition comprising adapalene and dapsone of the present disclosure.

According to an embodiment of the present disclosure, the skin disease is acne vulgaris, rosacea, rosacea, chronic wounds, bedsores, keratosis, sebaceous cysts, post-inflammatory hyperpigmentation, eczema, xerosis, pruritus, lichen planus, dermatitis, atopic dermatitis, prurigo nodularis, or prickly heat.

According to an embodiment of the present disclosure, the skin disease is acne vulgaris.

In the present invention, acne vulgaris may occur on the face, chest, back, and in some cases may be more widespread. Depending on the severity, acne vulgaris may be classified as mild, moderate, or severe. Mild acne is typically characterized by the presence of comedones (blackheads and whiteheads) and may also include papules and pustules. Moderate acne is generally characterized by more painful, deep, inflamed lesions, which may lead to scaring. Severe acne is characterized by deep, inflamed lesions, including cysts and nodules, which may be painful and may also lead to scarring.

The topical topical dermatological pharmaceutical composition disclosed herein has the following beneficial effects:
1) The present disclosure utilizes a combination of specific types of oily solvent, a surfactant, and a co-surfactant to prepare a clear, non-layering, transparent microemulsion system of a composition comprising adapalene and dapsone with excellent stability. In addition, the emulsion comprising adapalene and dapsone prepared based on the above the microemulsion system, when stored under conditions of 25±2°C/60±5% RH, 30±2°C/65%±5% RH, and 40±2°C/75%±5% RH, shows no significant changes in appearance, pH, viscosity, particle size, active ingredient content, and related substances before and after storage for at least 12 months, demonstrating excellent stability.
2) In the composition provided by the present disclosure, the solubility of adapalene in the composition vehicle is more than 100 times higher than its solubility in water, which is beneficial for improving the absorption of adapalene.
3) In the compositions provided by the present disclosure, the inventors, after extensive screenings, surprisingly discovered that when the weight ratio of the polyoxyethylene nonionic surfactant to the oily solvent is greater than 1:1, a clear, non-layering, and highly transparent microemulsion system can still be prepared even with relatively low amounts of the polyoxyethylene nonionic surfactant and the oily solvent. In addition, the stickiness, odor, and irritation of the system is reduced.
4) In the composition provided by the present disclosure, a crystallization inhibitor is incorporated, which significantly reduces the particle size of the drug particles and inhibits their growth of, thereby reducing a gritty sensation during application.
5) In the composition provided by the present disclosure, the oil droplet size is less than 300 nm, thereby facilitating the rapid dermal absorption of the active pharmaceutical ingredient.
6) In the composition provided by the present disclosure, the release rate of dapsone is increased by 1.5 times compared with the prior art, and the amount of absorption in the epithelium and dermis is increased by 1.9 times compared with the prior art, thereby improving therapeutic efficacy.
7) The composition provided by the present disclosure has a good therapeutic effect on diseases such as acne.
8) The composition provided by the present disclosure reduces the drug-induced skin irritation and exhibits good tolerance and safety.
9) The composition provided by the present disclosure is non-greasy and easy to spread.

### Brief Description of Drawings

Fig. 1 shows a polarized light microscopy (PLM) image of the E15 sample in Test Example 2.
Fig. 2 shows a polarized light microscopy (PLM) image of sample E2 in Test Example 2.
Fig. 3 shows a polarized light microscopy (PLM) image of the E11 sample in Test Example 2.
Fig. 4 shows a polarized light microscopy (PLM) image of the R2 sample in Test Example 2.
Fig. 5 shows an overlay of XRD patterns of samples E2, E11, E15, and R2 in Test Example 2.
Fig. 6 shows the percentage change from baseline in total ear lesions severity in each treatment group in Test Example 5 after 11 days and 14 days of dosing in rabbit ears.
Fig. 7 shows the percentage change from baseline in inflammatory ear lesion severity in each treatment group in Test Example 5 after 11 days and 14 days of of dosing in rabbit ears.

### Detailed Description

Hereinafter, the present disclosure will be described in further detail with reference to the following examples in order to better illustrate various aspects and advantages of the present dislcosure. However, it should be understood that the following examples are non-limiting and are provided merely to illustrate certain embodiments of the present disclosure.

In the present disclosure, an emulsion is an emulsified system formed by two immiscible phases through a specific emulsifier (surfactant), in which fine droplets of one liquid are uniformly dispersed in the other liquid, the former is referred to as the dispersed phase and the latter is referred to as the continuous phase. Conventionally, the organic liquid constituting the emulsion is referred to as "oil (O)". An oil (O) dispersed in water (W) is called an oil-in-water emulsion (O/W); whereas a water-in-oil emulsion (W/O) refers to the opposite system. To improve the solubility and transdermal absorption of hydrophobic drugs, the active pharmaceutical ingredient is dissolved in oil-phase droplets, which are then dispersed in the aqueous phase to form a homogeneous and stable O/W emulsion. When the oil droplet size is less than 300 nm, the emulsion is referred to as a microemulsion.

In the present disclosure, an emulgel or emulgel dosage form is a gel-based emulsion dosage form. A gel or gel dosage form refers to thick liquid or semi-solid preparation having gel characteristics, which is prepared from an active pharmaceutical ingredient and an excipient capable forming a gel. An emulgelrefer to a thick liquid or semi-solid preparation having gel characteristics, in which the active pharmaceutical ingredient is dissolved/dispersed in oil phase droplets, and the oil-phase droplets, the oil-phase droplets are further dispersed in gel-forming excipients.

In the present disclosure, cream or a cream dosage form refers to a homogeneous semi-solid topical preparation prepared by mixing an active pharmaceutical ingredient with oily or water-soluble base. Depending on the nature of the base, creams may be classified into oil-in-water creams and water-in-oil creams due to different creams.

In the present disclosure, a nonionic surfactant is a surfactant that does not ionize in water and is insensitive to changes in pH.

In the present disclosure, a "poorly water-soluble drug" refers to a drug known to be applicable in the pharmaceutical field and has low solubility in water relative to its effective dosage. Specifically, it refers to a drug requiring more than 100 mL of solvent (water) to dissolve 1 g or 1 mL of solute (corresponding to a concentration of 1% or less), preferably more than 1,000 mL (corresponding to a concentration of 0.1% or less), and more preferably more than 10,000 mL (corresponding to a concentration of 0.01% or less).

In this disclosure, "pharmaceutically acceptable carrier" refers to any pharmaceutically acceptable ingredient known to those skilled in the art, which is generally regarded as an inactive ingredient.

In the present invention, the content of adapalene is determined by HPLC. The mobile phase A is 0.05% TFA/acetonitrile (95%), and the mobile phase B iswater (5%). The detection wavelength is 270 nm.

### Screening Example 1: Screening of oily solvents, surfactants, and cosurfactants

### (1) Solubility of adapalene in various media.

Solubility test method: Take 2-3 mL of the medium in Table 1, add an excess of adapalene to obtain a suspension, stir at room temperature for 24 hours, filter, and take the clarified filtrate for testing.

In the present dislocure, asolubility of adapalene in a medium of about 400 µg/ml or higher is considered sufficient to meet the solubility requirement.

The results are shown in Table 1:

**Table 1**

| Classification | Medium | Solubility (µg/ml) |
|---|---|---|
| water | purified water | 0.07 |
| oily solvents | medium chain triglycerides | 223.3 |
| | diethyl sebacic acid | 551.3 |
| | isopropyl myristate | 184.6 |
| | diisopropyl adipate | 443.20 |
| | Tri acetin | 134.6 |
| | ethyl oleate | 120.06 |
| | propylene glycol monolaurate | 450.34 |
| | propylene glycol monocaprylate | 802.5 |
| surfactants | glyceryl monooleate | 398.6 |
| | polyethylene glycol glyceride caprylate | 1473.8 |
| | diethylene glycol monoethyl ether / Polyoxyethylene (35) castor oil (2/1)* | 3898.04 |
| | diethylene glycol monoethyl ether / polyoxyethylene (40) hydrogenated castor oil (2/1)* | 4454.9 |
| | diethylene glycol monoethyl ether / polyethylene glycol (15)-hydroxystearate (2/1)* | 4158.4 |
| co-surfactants | diethylene glycol monoethyl ether | 2216.7 |
| | ethanol | 128.6 |
| | Propylene glycol | 19.4 |

| | | |
|---|---|---|
| *Note: Polyoxyethylene (35) castor oil, polyoxyethylene (40) hydrogenated castor oil and polyethylene glycol (15)-hydroxystearate are solid or semi-solid at room temperature. They therefore need to be first dissolved in diethylene glycol monoethyl ether, and the solubility of adapalene in the mixed medium is then determined. | | |

The results in Table 1 show that, in oily solvents, the solubility of adapalene in propylene glycol monocaprylate, propylene glycol monolaurate, diisopropyl adipate and diethyl sebacic acid is all above 400 µg/ml and thus meet the solubility requirements.

In surfactants, the solubility of adapalene in diethylene glycol monoethyl ether/polyoxyethylene (35) castor oil (2/1), diethylene glycol monoethyl ether/polyoxyethylene (40) hydrogenated castor oil (2/1), and diethylene glycol monoethyl ether/polyethylene glycol (15)-hydroxystearate (2/1) is all above 3000 µg/ml; the solubility in polyethylene glycol glyceride caprylate is above 1000 µg/ml; and the solubility in monoolein is approximately 400 µg/ml. All of these meet the solubility requirements.

In co-surfactants, the solubility of adapalene in diethylene glycol monoethyl ether is above 2000 µg/ml and thus meets the solubility requirements.

### (2) Further screening of surfactant types

Using propylene glycol monocaprylate as the oily solvent and diethylene glycol monoethyl ether as the co-surfactant, the appearance, transparency, stability and solubility of adapalene in the systems formed with different surfactant combinations listed in Table 2 were evaluateded.

### Test method:

At room temperature, 5% propylene glycol monooctanoate, 30% diethylene glycol monoethyl ether, and 15% surfactant were added to a beaker and stirred until uniform. Purified water was then added dropwise to the beaker while stirring to make up to the total volume 100%, thereby forming a sample system. The following parameters were evaluated, and the results are shown in Table 2.
a) Appearance: the sample was allowed to stand and whether it is clear and whether phase separation occurs was observed.
b) Transparency: For samples showing no phase separation after being placed at room temperature for one day, the transparency of the composition was tested using UV-Vis spectroscopy (transmittance at 650 nm, with water as the blank).
c) Stability: For samples showing no phase separation after being placed at room temperature for one day, the sample was centrifuged at 8000 rpm for 15 min, and whether separation occurs was observed.
d) Adapalene solubility: 2-3 mL of sample was taken, andan excess amount of adapalene was added to obtain a suspension. The suspension was stirred at room temperature for 24 h, then filtered, and the clear filtrate was collected for analysis.

**Table 2**

| Surfactants | Characterization Results | | | |
|---|---|---|---|---|
| | Appearance | Transparency (%) | Stability | Solubility of Aadapalene (µg/mL) |
| Polyoxyethylene (35) castor oil | clear and transparent | 99.41 | no phase separation, no change in appearance | 400.41 |
| Polyoxyethylene (40) hydrogenated castor oil | clear and transparent | 99.88 | no phase separation and no change in appearance | 346.46 |
| Polyethylene glycol glyceride caprylate | turbid, milky white | 0.21 | no phase separation and no change in appearance | 151.51 |
| Monooleate glyceride | turbid, milky white | 0.03 | no phase separation and no change in appearance | N/A |
| Polyethylene glycol (15)-hydroxystearate | turbid, milky white | 0 | phase separated | N/A |

The results in Table 2 show that when propylene glycol monocaprylate is used as the oily solvent, and diethylene glycol monoethyl ether is sued as the co-surfactant, and polyoxyethylene (35) castor oil or polyoxyethylene (40) hydrogenated castor oil is selected as the surfactant, the obtained samples are clear and transparent, with a transparency of more than 99%, good phase stability, low tendency for oil-water separation, and high solubility for adapalene. while when polyethylene glycol glyceride caprylate, monooleate glyceride or polyethylene glycol (15)-hydroxystearate are selected as surfactants, the obtained samples are turbid, indicating larger droplet size, higher tendency for oil-water separation, and a risk of poor stability.

### (3) Further screening of oily solvent types

Using diethylene glycol monoethyl ether as a co-surfactant and polyoxyethylene (35) castor oil as the surfactant, the appearance, clarity, stability and solubility of adapalene in systems formed with different oily solvents shown in Table 3 were evaluated.

### Test method:

At room temperature, 15% polyoxyethylene (35) castor oil, 30% diethylene glycol monoethyl ether, and 5% oily solvent were added into a beaker and stirred until uniform. Purified water was added dropwise to the beaker while stirring to make up to 100% to form a sample system. The sample was evaluated according to the following parameters.
a) Appearance: the sample was allowed to stand and whether it is clear and whether phase separation occurs was observed.
b) Transparency: For samples showing no phase separation after being stored at room temperature for one day, the transparency of the sample was tested using UV-Vis spectroscopy (transmittance at 650 nm, with water as the blank).
c) Stability: For samples showing no phase separation after being placed at room temperature for one day, the sample was centrifuged at 8000 rpm for 15 min, and whether separation occurs was observed.
d) Adapalene solubility: 2-3 mL of different samples were taken, and an excess amount of adapalene was added to obtain a suspension. The suspension was stirred at room temperature for 24 h, filtered, and the clear filtrate was collected for analysis.

The results are shown in Table 3.

**Table 3**

| Oily Solvents | Sample Characterization Results | | | |
|---|---|---|---|---|
| | Appearance | Transparency (%) | Stability | Solubility of Adapalene (µg/mL) |
| Propylene glycol monocaprylate | transparent clear | 99.41 | no phase separation, no change in appearance | 400.41 |
| Propylene glycol monolaurate | transparent clear | 96.33 | no phase separation, no change in appearance | 414.97 |
| Diisopropyl adipate, diethyl sebacate | milky white, turbid | 0.16 | phase separated | N/A |
| Diethyl sebacate | milky white, turbid | 0 | phase separated | N/A |
| Medium-chain triglycerides | milky white, turbid | 0 | turbid, milky white | N/A |
| Soybean oil | milky white, turbid | 0 | turbid, milky white | N/A |

The results in Table 3 show that when diethylene glycol monoethyl ether is the cosurfactant and polyoxyethylene (35) castor oil is the surfactant, and propylene glycol monocaprylate and propylene glycol monolaurate are selected as the oily solvents, respectively, the obtained samples are transparent and clear, good stability, a transparency of more than 96%, and a high solubility for adapalene. While when diisopropyl adipate, diethyl sebacate, medium-chain triglycerides or soybean oil are used as oily solvents, the obtained samples are turbid, indicating larger droplet size, higher tendency for oil-water separation, and a risk of poor stability.

### Screening Example 2: Investigation of the ratio betweenoily solvent and nonionic surfactant

Based on the results of screening example 1, in this screening example used propylene glycol monocaprylate was usded as the oily solvent, diethylene glycol monoethyl ether was used as the co-surfactant, and polyoxyethylene (35) castor oil was used as the surfactant to prepare a composition comprising adapalene and dapsone. The effects of compositions formed with different ratios of oily solvent and surfactant on the droplet appearance, clarity, stability and the solubility of adapalene were investigated.

### Test method:

At room temperature, according to the formulation in Table 4, adapalene, dapsone, propylene glycol monocaprylate, polyoxyethylene (35) castor oil and diethylene glycol monoethyl ether were mixed and stirred until uniform to obtain an oil phase solution. Purified water was then added to the oil phase solution, and was stirred to homogeneity, thereby preparing a composition comprising adapalene and dapsone.

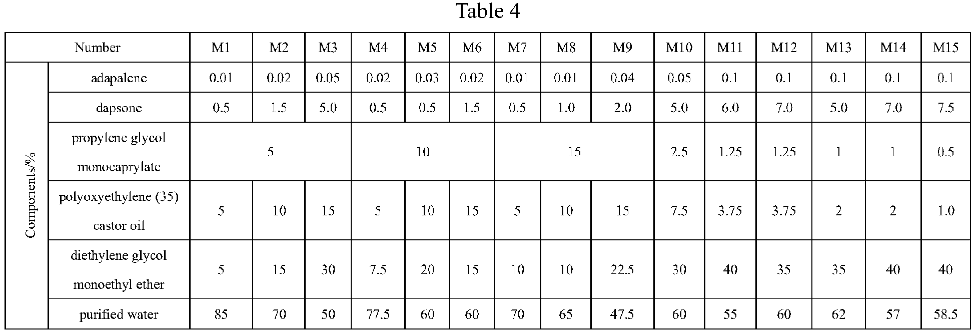

The composition samples prepared above were evaluated using the following parameters.

Appearance: the sample was allowed to stand and whether it is clear and whether phase separation occurs was observed.

Stability: For samples showing no phase separation after being placed at room temperature for one day, the sample was centrifuged at 8000 rpm for 15 min, and whether separation occurs was observed.

Transparency: For samples showing no phase separation after being stored at room temperature for one day, the transparency of the sample was tested using UV-Vis spectroscopy (transmittance at 650 nm, with water as the blank).

Droplet size: A sample of the composition was taken and measured three times using a laser particle size analyzer, and the average value of the droplet size was calculated.

pH: A sample of the composition was taken and measured three times using a pH meter, and its average pH value was calculated.

The results are shown in Table 5.

**Table 5**

| Number | M1 | M2 | M3 | M4 | M5 | M6 | M 7 | M8 | M9 | M10 | M11 | M12 | M1 3 | M14 | M15 |
|---|---|---|---|---|---|---|---|---|---|---|---|---|---|---|---|
| Appearance | phase separated | clear, colorless | | Phase separated | clear, colorless | | phase separated | | clear, colorless | | | | | | |
| Stability | phase separated | no phase separation, no unchanged | | phase separated | no phase separation, no change in appearance | | phase separated | | no phase separation, no unchanged in appearance | | | | | | |
| | | in appearance | | | | | | | | | | | | | |
| Transparency /% | 0 | 94.1 | 99.6 | 0 | 97.1 | 99.7 | 0 | | >99 | | | | | | |
| Oil droplet size /nm | / | 48.9 | 58.2 | / | 158. 4 | 88.0 | / | / | 241.1 | 50.2 | 46.4 | 42.6 | 46.5 | 52.5 | 53.1 |
| pH | / | 6.0 | 6.5 | / | 6.3 | 5.9 | / | / | 6.0 | / | / | / | / | / | / |

The results in Table 5 show that: 1) when the weight ratio of polyoxyethylene (35) castor oil/propylene glycol monocaprylate is greater than 1:1, such as 1.5:1, 2:1 or 3:1 (M2, M3, M6, M10-M15), the resulting compositions are colorless and transparent microemulsions with an oil droplet sizes of less than 100 nm and exhibit good stability, with no turbidity or phase separation observed.

2) When the weight ratio of polyoxyethylene (35) castor oil/propylene glycol monocaprylate is 1:1 and each of polyoxyethylene (35) castor oil and propylene glycol monocaprylate is present at 15% or 10% by weight of the composition (M9 and M5), the resulting compositions are colorless and transparent microemulsion with oil droplet sizes of less than 250 nm, and good stability with no turbidity or phase separation observed.

3) When the weight ratio of polyoxyethylene (35) castor oil/propylene glycol monocaprylate is 1:1, and each of polyoxyethylene (35) castor oil and propylene glycol monocaprylate is present at 5% (M1), the resulting composition is milky white and phase separation observed during the storage process.

4) When the weight ratio of polyoxyethylene (35) castor oil/propylene glycol monocaprylate is less than 1:1 (M4, M7, M8), the resulting compositions are milky white and phase separation observed during storage.

The results in Table 5 show that: 1) when the weight ratio of polyoxyethylene (35) castor oil/propylene glycol monocaprylate is greater than 1:1, such as 1.5:1, 2:1 or 3:1 (M2, M3, M6, M10-M15), the resulting compositions are colorless and transparent microemulsion with oil droplet sizes of less than 100 nm and good stability with no turbidity or phase separation observed.

In summary, when the weight ratio of polyoxyethylene (35) castor oil/propylene glycol monocaprylate is greater than 1:1, or when the mass ratio of polyoxyethylene (35) castor oil/propylene glycol monocaprylate is 1:1 while maintaining a relatively high content of surfactant, a composition comprising adapalene and dapsone with good stability, high transparency and small oil droplet size can be obtained. In contrast, when the weight ratio of polyoxyethylene (35) castor oil to propylene glycol monocaprylate is 1:1 and the surfactant content is relatively low, or when the weight ratio of polyoxyethylene (35) castor oil to propylene glycol monocaprylate is less than 1:1, the resuling composition is milky white and turbid, and exhibited phase separation, and has poor stability.

### Example 1: Preparation of a composition comprising adapalene and dapsone (emulsion)

The emulgel of the present inventions were prepared according to theformulatuons shown in Table 6 and Table 7.

**Table 6**

| Number | E1 | E2 | E3 | E4 | E5 | E6 | E7 | E8 | E9 | E10 | E11 |
|---|---|---|---|---|---|---|---|---|---|---|---|
| Adapalene | 0.1% | | | | 0.2% | | | 0.3% | | | 0.1% |
| Dapsone | 7.5% | 7.5% | 6% | 5% | 7.5% | 6% | 5% | 7.5% | 6% | 5% | 7.5% |
| Propylene glycol monocaprylate | 0.25% | 0.5% | | | | | | | | | |
| Olyoxyethylene (35) castor oil | 0.5% | 1% | | | | | | | | | |
| Diethylene glycol monoethyl ether | 40% | | | | | | | | | | |
| Pemulen TR-1 | 0.2% | | | | | | | | | | |
| Carbomer 981 | 0.2% | | | | | | | | | | |
| SEPINEO P 600 | 1.5% | | | | | | | | | | |
| PVP-K30 | 2% | | | | | | | | | | 0% |
| Methyl hydroxybenzoate | 0.2% | | | | | | | | | | |
| Butylated hydroxytoluene | 0.1% | | | | | | | | | | |
| Disodium edetate | 0.005% | | | | | | | | | | |
| Sodium hydroxide | Adjust the pH to 5.0 to 6.0 | | | | | | | | | | |
| Purified water | Make up to 100% | | | | | | | | | | |

**Table 7**

| Number | E12 | E13 | E14 | E15 | E16 | E17 | E18 | E19 | E20 | E21 |
|---|---|---|---|---|---|---|---|---|---|---|
| Adapalene | 0.1% | | | | | | 0.075% | 0.15% | 0.3% | 0.3% |
| Dapsone | 7.5% | | | | 6% | 5% | 3% | 6% | 12% | 7.5% |
| Propylene glycol monocaprylate | 1% | 1.25% | 2.5% | 5% | | | | | | |
| Olyoxyethylene (35) castor oil | 2% | 3.75% | 7.5% | 15% | | | | | 10% | 15% |
| Diethylene glycol monoethyl ether | 40% | 30% | | | | | | | 40% | 30% |
| Pemulen TR-1 | 0.2% | | | | | | | | | |
| Carbomer 981 | 0.2% | | | | | | | | | |
| SEPINEO P 600 | 1.5% | | | | | | | | | |
| PVP-K30 | 2% | | | | | | | | | |
| Methyl hydroxybenzoate | 0.2% | | | | | | | | | |
| Butylated hydroxytoluene | | | | 0.1% | | | | | | |
| Disodium edetate | | | | 0.005% | | | | | | |
| Sodium hydroxide | | | | Adjust pH to5.0-6.0 | | | | | | |
| Purified water | | | | Make up to 100% | | | | | | |

According to the formulations in Tables 6 and 7:
a) Preparation of aqueous phase:
   An appropriate amount of purified water was added to a water-phase vessel, and disodium edetate was added thereto and stirred until completely dissolved. Carbomer 981 and Pemulen TR-1 were then added, and the mixture was stirred until uniformly dispersed to obtain an aqueous-phase solution.
b) Preparation of oil phase:
   Diethylene glycol monoethyl ether was added to an oil-phase vessel, followed by the addition of PVP-K30, methyl paraben, and butylated hydroxytoluene (BHT), and the mixture was stirred until completely dissolved. Dapsone was then added and stirred until completely dissolved to obtain oil-phase solution 1 containing dapsone.
   Polyoxyethylene (35) castor oil was added to a separate oil-phase vessel and heated under stirring at 50-60°C to form a clear yellow liquid. Propylene glycol monocaprylate was then added, and the mixture was stirred until uniformly mixed, thereby obtaining oil-phase solution 2.
   Oil-phase solution 2 was added to oil-phase solution 1, and the mixture was stirred until uniform. Adapalene was then added and stirred to obtain a suspension oil-phase solution containing the active ingredient(s).
c) Preparation of sodium hydroxide solution
   Sodium hydroxide and an appropriate amount of purified water were mixed and stirred until completely dissolved.
d) Mixing and emulsification
   The aqueous-phase solution was added to the suspension oil-phase solution, and the mixture was stirred and homogenized. A sodium hydroxide solution and SEPINEO^{®} P 600 were then added, and the mixture was further stirred and homogenized to obtain the emulgel of the present disclosure.

### Comparative Example 1: Preparation of gel

Adapalene gel R1 was prepared according to Example 1 of US7838558, and dapsone gel R2 was prepared according to Example 1 of CN105246457B. The specific ormulations are shown in Table 8.

**Table 8**

| Number | R1 | R2 |
|---|---|---|
| Adapalene | 0.1% | / |
| Dapsone | / | 7.5% |
| Diethylene glycol monoethyl ether | / | 30% |
| Propylene glycol | 4% | / |
| Phenoxyethanol | 0.25% | / |
| Methyl parahydroxybenzoate | 0.1% | 0.2% |
| Carbomer | 1.1% | / |
| SEPINEO P 600 | / | 4% |
| Edetate disodium | 0.1% | / |
| Poloxamer | 0.2% | / |
| Sodium hydroxide | Adjust the pH to 5.0-6.0 | |
| Water | Make up to 100% | |

### Preparation method of:

According to the formulation shown in Table 8, phenoxyethanol, methyl parahydroxybenzoate, poloxamer, and edetate disodium were added to water and dissolved, and then carbomer was added and mixed uniformly to obtain an aqueous phase. Adapalene was added to propylene glycol and mixed uniformly to obtain an organic phase. Under stirring, the organic phase was slowly added to the aqueous phase, uniformly dispersed and homogenized to obtain the adapalene hydrogel of Comparative Example 1.

### Preparation method of Comparative Example R2:

According to the formulation shown in Table 8, methyl parahydroxybenzoate was added to water and dissolved to obtain an aqueous phase. Aapsone was added to diethylene glycol monoethyl ether and mixed uniformly to obtain an organic phase. Under stirring, the organic phase was slowly added to the aqueous phase, and finally SEPINEO P 600 was added and uniformly dispersed and homogenized to obtain the dapsone hydrogel of Comparative Example 2.

### Test Example 1: Comparative study of in vitro release and skin absorption.

The in vitro release and skin absorption of dapsone in the emulsions provided by the present disclosure (Examples E2 and E3) were compared with those of the prior art hydrogel (Comparative Example R2).

### Test method:

In vitro release: A vertical diffusion cell was used A solution of isopropyl alcohol: cyclodextrin: potassium dihydrogen phosphate:water (30:5:0.6:64.4) was used as the receiving medium for dapsone. A PVDF was used as the filter membrane and fixed between the donor chamber and the receptor chamber of the diffusion cell. The diffusion cell was mounted in a transdermal absorption diffusion apparatus. The in vitro release tests were conducted at 32°C and 600 rpm. The dapsone content in the diffusion cell was determined by HPLC, and the release rate of dapsone within 6 hours was calculated.

Skin absorption: A vertical diffusion cell was used. A solution of isopropyl alcohol: cyclodextrin: potassium dihydrogen phosphate: water (30:5:0.6:64.4) was used as the receptor medium for dapsone. Porcine skin was used as the filter membrane and fixed between the donor chamber and the receptor chamber of the diffusion cell. The diffusion cell was mounted in a transdermal absorption diffusion appartus, and the skin absorption experiment was carried out at 32°C and 600 rpm. After 24 hours, the porcine skin was removed, and the residual formulation on the surface of the skin was wiped off with cotton swabs. The stratum corneum was then removed by the tape stripping, and the remaining epithelial and dermal tissues were cut into pieces. The dapsone in the tissues was extracted with methanol. The dapsone content was determined by HPLC, and the total absorption of dapsone in the epithelial and dermal tissues was calculated.

The results are shown in Table 9.

**Table 9**

| Sample | Release rate (µg/cm²/h^{1/2} | Absorption in the epithelium and dermis (µg/cm²) |
|---|---|---|
| Example E2 | 2761 | 7.5 |
| Example E3 | 3294 | 9.2 |
| Comparative Example R2 | 2223 | 4.9 |

The results in Table 9 show that the in vitro release rate of dapsone from the emulsion of the present disclosure is significantly higher than that from the hydrogel of the prior art. In particular, the in vitro release rates of dapsone in Examples E2 and E3 are 1.2 times and 1.5 times that of Comparative Example R2, respectively. The skin absorption of dapsone from the emulgels of the present disclosure is significantly higher than that from the hydrogel. Specifically, the absorption of dapsone in the epithelium and dermis in Examples E2 and E3 is 1.9 times and 1.5 times that of Comparative Example R2, respectively.

### Test Example 2: Comparative study of particle Size and dispersion State

Optical microscopy (PLM) and X-ray diffractometer (XRD) were used to compare the particle size and dispersion state of the crystal particles in the emulgel of the present disclosure (Examples E2, E11, E15) and the prior art hydrogel (Comparative Example R2).

### Test method:

The PLM images were acquired using an XPV-990E polarized light microscope. A small amount of sample was placed on a glass slide, covered with a cover slip, and placed on a stage for observation and photography. The particle size was subsequently statistically analyzed.

XRD data were collected using a Bruker D8 Advance diffractometer. Parameters were as follows: Cu target, wavelength 1.54Å, Current and voltage 40kV, 40mA, and a scanning rangeof 3 to 40° 2θ.

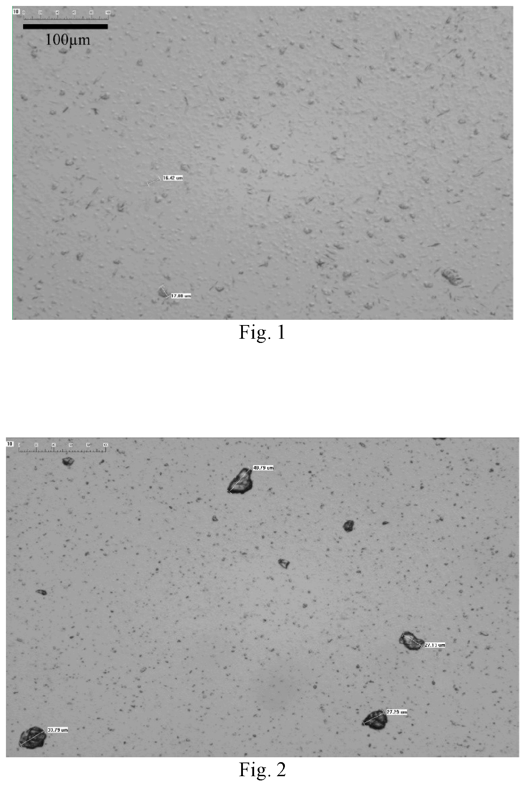

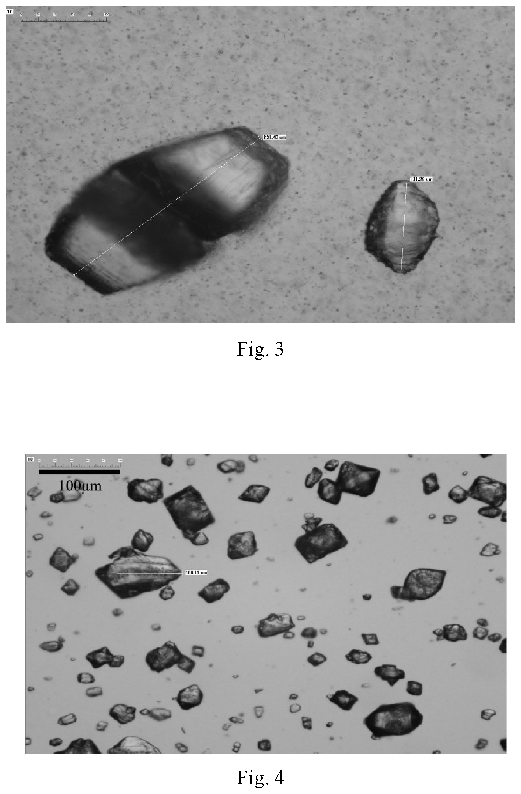

The results are shown in Figures 1-5.

The results in Figures 1-4 show that in the emulgels examples E2 and E15 provided in the present disclosure containing PVP-K30, the drug particle size was relatively small (D90 <30 µm), and no large particles exceeding 50 µm were observed. In the emulgel example E11 provided by the present disclosure without PVP-K30, a small amount of large particles exceeding 100 µm was observed. In the prior art dapsone hydrogel preparation R2 without PVP-PK30, a large number of particles exceeding 50 µm and 100 µm were observed.

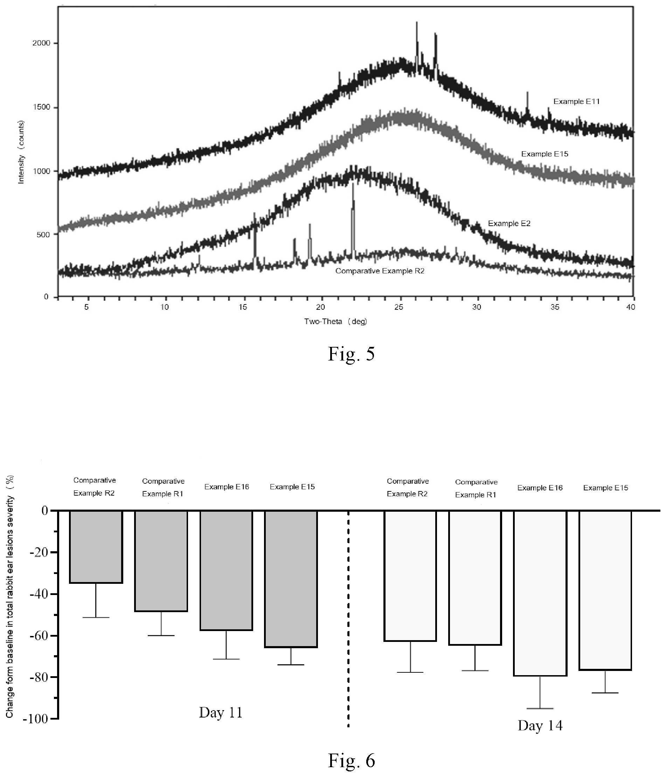

The results in Figure 5 show that no crystalline diffraction peaks were observed in the emulgel examples E2 and E15 containing PVP-K30 of the present disclosure, indicating that the drug particles in the formulations were small in size and were well dispersed. In the emulgel of the present disclosure without PVP-K30 (Example E11), crystalline diffraction peak were observed, but the peak intensity was weak. In contrast, in the prior-art dapsone hydrogel without PVP-K30 (Comparative Example R2), strong dapsone crystalline diffraction peaks were observed, indicating that the dapsone particles in the hydrogel were large in size and poorly dispersed.

### Test Example 3: Skin touch test

Since particle size in topical formulations may affect the sensory experience during application, the inventors of the present disclosure further evaluated the skin feel upon application of the emulgels of the present disclosure (Examples E2, E11 and E15) and the hydrogel of the prior art (Comparative Example R2).

The results show that in the emulgel Examples E2 and E15 of the present disclosure containing PVP-K30, no a gritty sensation was observed during application. The emulgel Example E11 of the present disclosure without PVP-K30 exhibited a slight gritty sensation, whereas the prior-art Comparative Example R2 of the dapsone hydrogel without PVP-K30 exhibited a significant gritty sensation during application.

The incorporation of PVP-K30 into the combination composition of adapalene and dapsone can inhibit growth of drug particles, reduce the particle size, and decrease the gritty sensation during application.

### Test Example 4: Stability study

The emulgel of the present disclosure was subjected to a long-term (25±2°C/60±5%RH) stability test. The following parameters were evaluated: appearance, pH, PLM particle size, viscosity, HPLC assay, and related substances. The results are shown in Tables 10-14.

**Table 10**

| Storage condition | Duration | E2 | E3 | E15 |
|---|---|---|---|---|
| | 0 month | white emulgel | white emulgel | white emulgel |
| | 1 month | white emulgel | white emulgel | white emulgel |
| Long-term (25+2°C/60+5%RH) | 3 months | white emulgel | white emulgel | white emulgel |
| | 6 months | white emulgel | white emulgel | white emulgel |
| | 9 months | white | white | white |
| | | emulgel | emulgel | emulgel |
| | 12 months | white emulgel | white emulgel | white emulgel |

The results in Table 10 show that the appearance of the emulgels of Examples E2, E3 and E15 remained unchanged for 12 months when stored at 25+2°C/60+5%RH, indicating good stability.

**Table 11**

| Storage condition | Emulgels | Duration | pH |
|---|---|---|---|
| | E2 | 0 month | 5.7 |
| | | 1 month | 5.80 |
| Long-term (25±2°C/60±5%RH) | E3 | 0 month | 5.7 |
| | | 1 month | 5.80 |
| | E15 | 0 month | 6.0 |
| | | 1 month | 6.0 |

The results in Table 11 show that after storage of the emulgels of Examples E2, E3 and E15 at 25±2°C/60±5%RH for 1 month, no significant change in pH of was observed, indicating good stability.

**Table 12**

| Storage condition | Emulgel | Duration | Viscosity/mPa·s |
|---|---|---|---|
| Long-term (25±2°C/60±5%RH) | E15 | 0 month | 23600 |
| | | 1 month | 24800 |

Viscosity test: Method 1: Test using a Brookfield viscometer with a TC (93) rotor, a rotation speed of 5 rpm, and a test time of 1 minute. Method 2: Test using a Brookfield viscometer with a TD (94) rotor, a rotation speed of 40 rpm, and a test time of 1 minute.

The results in Table 12 show that the viscosity of the emulgel of Example E15 remained essentially unchanged after storage at 25±2°C/60±5%RH for one month, indicating good stability.

**Table 13**

| Storage condition | Emulgel | Duration | Particle size (D90)/ µm |
|---|---|---|---|
| Long-term (25±2°C/60±5%RH) | E3 | 0 month | 5 |
| | | 1 month | 5 |
| | | 6 months | 4 |

The results in Table 13 show that the particle size (D90) of the emulgel of Example E3 remains essentially unchanged after storage at 25±2°C/60±5%RH for 6 months.

**Table 14**

| Emulgels | Storage condition | | Parameters | | | |
|---|---|---|---|---|---|---|
| | Temperature-humidity | Duration | Content /%^{[1]} | | Related substances /%^{[2]} | |
| | | | adapalene | dapsone | adapalene | dapsone |
| E2 | 25±2°C/60± 5%RH | 0 month | 101.4 | 100.2 | N.D. | N.D. |
| | | 3 months | 103.3 | 100.6 | N.D. | 0.06 |
| | | 6 months | 98.5 | 103.0 | <0.05 | <0.05 |
| | | 12 months | 102.9 | 103.9 | <0.05 | <0.05 |
| E3 | 25±2°C/60± 5%RH | 0 month | 100.2 | 103.8 | N.D. | N.D. |
| | | 3 months | 101.6 | 103.8 | N.D. | 0.07 |
| | | 6 months | 99.3 | 104.3 | <0.05 | 0.05 |
| | | 12 months | 102.2 | 102.4 | <0.05 | 0.09 |
| E15 | 25±2°C/60± 5%RH | 0 month | 100.6 | 100.4 | <0.05 | <0.05 |
| | | 3 months | 100.4 | 100.8 | 0.05 | <0.05 |
| | | 6 months | 99.2 | 100.5 | <0.05 | <0.05 |
| | | 12 months | 98.7 | 103.4 | <0.05 | <0.05 |

| | | | | | | |
|---|---|---|---|---|---|---|
| Notes: [1] Percentage of the labeled claim of the product; [2] Percentage of total impurities. | | | | | | |

The results in Table 14 show that after storage of the emulgels of Examples E2, E3 and E15 at 25±2°C/60±5%RH for 12 months, the assay of the active ingredients and the levels of related substances remained substantially unchanged, indicating good stability.

In summary, the emulgel prepared according to the present disclosure are white emulgels. Upon storage at 25±2°C/60±5%RH, its appearance, pH, viscosity, drug particle size (D90), assay of the active ingredient and the levels of related substance remain unchanged for at least 12 months. All quality attributes meet the product quality specifications, demonstrating good stability.

In addition, the inventors of the present disclosure also studied the storage stability of the emulgel (Examples E2, E3 and E15) under 40±2°C/75%±5%RH and 30±2°C/65%±5%RH conditions and also showed good stability.

### Test Example 5: Comparative study of drug efficacy

A rabbit ear acne model was used to compare the therapeutic effects of the emulgel of the present disclosure (Examples E15 and E16), the adapalene hydrogel of the prior art (Comparative Example R1), and a prior art dapsone hydrogel (Comparative Example R2) in the treatment of acne.

### Test method:

Model establishment: 2% coal tar was applied daily to a 2 cm x 2 cm area surrounding the opening of the right ear canal on the inner surface of New Zealand White rabbits at a dose of 0.5-1 ml per rabbit for 18 consecutive days. Starting on the eighth day, Cutibacterium acnes was injected intradermally into the right ear at 7-8 sites per rabbit, 30 µL/site, every other day for a total of 6 injections. Twenty-four hours after the final injection, all rabbit ears were photographed and observed, and the number of comedones, papules, and pustules were counted.

Grouping: After the model establishment, animals were randomly divided into a model group, an Example E15 administration group, an Example E16 administration group, a Comparative Example R1 administration group, and a Comparative Example R2 administration group.

Administration: After grouping, treatment was initiated. The test formulation was applied to the rabbit ear lesion site at a dose of 0.4 mL per rabbit per day for 2 consecutive weeks.

The numbers of comedones, papules and pustules in each group were counted before administration (Day 0), and on 11 days and 14 days after administration.

Twenty-four hours after the last administration, the right ear was collected and fixed with 4% paraformaldehyde. The ears were routinely paraffin-embedded and sectioned. Hematoxylin and eosin (H&E) staining was performed, and the histopathological morphological changes of the ear tissues were observed under a light microscope (including general observation parameters: microscope (including general observation parameters: hyperkeratosis, acanthosis, follicular dilation, sebaceous gland hyperplasia, inflammatory cell infiltration, and histological evaluation of experimental comedones).

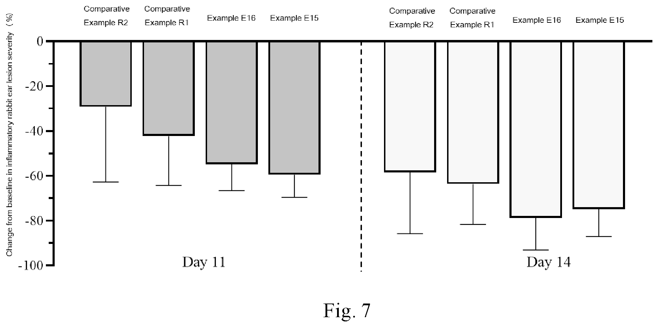

### The results are shown in Figures 6 and 7.

The results showed that after 11 and 14 days of administration, the percent change of total ear skin lesions (comedones + papules + pustules) and the percent change of inflammatory ear skin lesions (papules + pustules) compared to the baseline in the emulgels of the present disclosure (Examples E15 and E16) were lower than those in the dapsone hydrogel (Comparative Example R2) and the adapalene hydrogel (Comparative Example R1), indicating that the emulgels of the present disclosure provide superiot therapeutic effecacy on both total acne lesions and inflammatory skin lesions compared with the dapsone hydrogel and adapalene hydrogel of the prior art.

In addition, in terms of hispathological structure, 14 days after administration, the emulgel of the present disclosure (Examples E15 and E16) showed superior effects to the prior art dapsone hydrogel (Comparative Example R2) and adapalene hydrogel (Comparative Example R1) in improving hyperkeratosis of the rabbit ear epidermis, keratinous material accumulation, folliclar dilation, sebaceous gland hyperplasia, and inflammatory cell infiltration.

### Test Example 6: Toxicity study

The toxicity of the emulgels Example E19 (an emulsion comprising 0.15% adapalene and 6% dapsone) and Example E20 (an emulgel comprising 0.30% adapalene/12% dapsone) of the present disclosure was studied.

### (1) Guinea pig active skin sensitization test

### Test method:

Grouping: British guinea pigs were randomly divided into a negative control group, a positive control group, a blank emulgel control group (without adapalene and dapsone, with the remaining components identical to those of administration group), an Example E19 administration group, and an Example E20 administration group.

### Sensitization administration

Preparation before administration: Before administration, the hair on the left dorsal skin of the guinea pig was removed, with a depilated area of approximately 5 cm × 5 cm.

On the 1st, 8th and 15th days of the study, the negative control group was administered sterile water for injection dermally at a dose of 0.2 mL per animal, the positive control group was topically applied with 0.2 mL per animal 1% 1-chloro-2,4-dinitrobenzene. The remaining groups were each topically applied with the corresponding test substance at a dose of 0.5 g per animal. The treated area were covered with cellophane (approximately 4 cm × 4 cm) and gauze and fixed with medical adhesive tape for about 6 hours before removal of the dressing.

### Challenge administration

Preparation before administration: Before administration, the hair on the right dorsal side of the guinea pig was removed, with a depilated area approximately 5 cm × 5 cm;

On the 29th day of the study, the negative control group was administered sterile water for injection dermally at a dose of 0.2 mL pera nimal, the positive control group was topically applied with 0.2 mL per animal 0.5% 1-chloro-2,4-dinitrobenzene , and the remaining groups were each topically applied with the corresponding test substance at a dose of 0.5 g per animal. The tested area was covered with cellophane (approximately 4 cm × 4 cm) and gauze, and fixed with medical adhesive tape for approximately 6 hours before removal of the dressing.

### Observation and scoring

Observation time: 1 hour (±30 minutes), 24 hours (±2 hours) after each sensitization administration and 1 hour (±30 minutes), 24 hours (±1 hour), 48 hours (±2 hours), and 72 hours (±2 hours) after each challenge administration.

### Animals observed: All surviving guinea pigs in each group;

Observation parameters: The skin at the administration site was visually examined and scored. The total score for each skin site at each time point was calculated, and the mean skin observation score for each dose group. After challenge administration, animals were observed for systemic allergic reactions such as asthma, unsteady posture, or shock.

The results showed that, except for the positive control drugs, no obvious skin and systemic allergic reactions observed in the guinea pigs in each group, indicating that the emulgel agent disclosed of the present disclosure is non-sensitizing.

### (2) Long-term toxicity study in miniature pigs

### Test method:

Grouping: Bama miniature pigs were randomly divided into a blank control group, a blank emulgel control group (without adapalene and dapsone, with the remaining components identical to the drug-administered group), an Example E19 administered group, and an Example E20 administered group.

### Dosage

The formulation was applied topically to the dorsal skin once daily for 28 consecutive days.

Preparation before dosing: Before administration, the dorsal hair of the miniature pigs was removed, with the depilated area slightly larger than the application area for each animal. The depilation frequency was at least once a week. Before administration, the application sites were marked with a marker pen and the skin condition was carefully observed to avoid skin damage. Every 2 to 28 days prior to administration, the application sites were cleaned with drinking water. and the skin of the test area was wiped dry with gauze or a clean towel before administration.

Administration: A required amount of the test article was weighed and uniformly applied to the depilated dorsal area of each miniature pig. The dose and application area for each animal were calculated based on the most recent body weight. The application area was calculated as: application area = dose ÷ dose per unit area.

The results showed that, during the administration period, all pigs in each group were in good condition, with normal spontaneous activities, and no death or moribund state was observed, and no other obvious toxic symptoms observed. The irritation scores of the administration sites in all the pigs in each group were 0, and no abnormal manifestations such as erythema or edema were observed, indicating no irritation to the skin at the administration site. No obvious abnormal changes were observed in the body weight or food consumption of the pigs in any group. At the end of the administration, no obvious abnormal changes were observed in hematological parameters in any group, including WBC and differential counts and percentages, RBC, HGB, HCT, MCV, MCH, MCHC, PLT, RET, RET%, APTT, and PT, etc. No obvious abnormal changes in the ALT, AST, ALP, LDH, GGT, UREA. No obvious abnormal changes were observed in the serum biochemical parameters in any group, including CREA, CHOL, TP, ALB, GLB, TG, TBIL, CK, GLU, A/G, K+, Na+, and Cl-. No obvious abnormal changes were observed in organ weights and organ-to-body and organ-to-brain weight ratios in any group, including brain, liver, kidney, adrenal gland, heart, spleen, thyroid gland, thymus/parathyroid gland, epididymis, testis, ovary, and uterus/cervix. Gross necropsy examination of other organs or tissues of the pigs in each group showed no test article-related abnormal changes in size, shape, color, and texture. No abnormal histopathological changes related to the test article were observed in the skin at the administration site or in the surrounding subcutaneous tissues in any group.

### Test Example 7: User Experience Comparison

### Test method:

This study was conducted in 10-20 healthy subjects to evaluate functional and cosmetic properties. The test products (Example E2, Comparative Example R2) were identified using blind identification codes to prevent the subjects from knowing the characteristics of the applied test formulations. The formulations were randomly applied to their left and right arms. The age range of the subject population was between 20 and 40 years old.

The following properties were evaluated during and after application, ease of application, foreign body sensation during application, tackness after application, odor, rate of absorption after application, drying time of the skin, skin feel after application, overall cosmetic preference and usability of the product.

The results showed that all subjects reported no significant difference in application experience between Example E2 and Comparative Example R2. In addition, the subjects generally indicateed that Example E2 had advantages of good spreadability no foreign-body sensation, non-tacky feel, no unpleasant odor, and rapid absorption.

## Claims

1. A composition comprising adapalene and dapsone, wherein based on the total weight of the composition, the composition comprises 1%-15% of dapsone, 0.05%-0.5% of adapalene, 0.25%-20% of carboxylic acid ester-based oily solvent, 0.5%-20% of polyoxyethylene nonionic surfactant, 5%-50% of diethylene glycol monoethyl ether, and other pharmaceutically acceptable carriers.

2. The composition according to claim 1, wherein based on the total weight of the composition, the composition comprises 0.25-15% of carboxylic acid ester-based oily solvent.

3. The composition according to claim 2, wherein based on the total weight of the composition, the composition comprises 0.25-5% of carboxylic acid ester-based oily solvent.

4. The composition according to claim 3, wherein based on the total weight of the composition, the composition comprises 0.25-1% of carboxylic acid ester-based oily solvent.

5. The composition according to claim 1, wherein the carboxylic acid ester-based oily solvent is selected from any one of monocarboxylic acid esters, dicarboxylic acid esters, and tricarboxylic acid esters, or a mixture thereof.

6. The composition according to claim 5, wherein the carboxylic acid ester-based oily solvent is a monocarboxylic acid ester-based oily solvent.

7. The composition according to claim 5 or 6, wherein the monocarboxylic acid ester-based oily solvent is selected from any one of propylene glycol monocaprylate and propylene glycol monolaurate, or a mixture thereof.

8. The composition according to any one of claims 5-7, wherein the carboxylic acid ester-based oily solvent is propylene glycol monocaprylate.

9. The composition according to claim 1, wherein based on the total weight of the composition, the composition comprises 0.5%-15% of polyoxyethylene nonionic surfactant.

10. The composition according to claim 9, wherein based on the total weight of the composition, the composition comprises 0.5%-10% of polyoxyethylene nonionic surfactant.

11. The composition according to claim 10, wherein based on the total weight of the composition, the composition comprises 0.5%-5% of polyoxyethylene nonionic surfactant.

12. The composition according to claim 1, wherein the polyoxyethylene nonionic surfactant is selected from any one of polyoxyethylene castor oil, fatty alcohol polyoxyethylene ether, polyol polyoxyethylene ether fatty acid ester, alkylphenol polyoxyethylene ether, fatty acid polyoxyethylene ester, and fatty amine polyoxyethylene ether, or a mixture thereof.

13. The composition according to claim 12, wherein the polyoxyethylene nonionic surfactant is selected from polyoxyethylene castor oil.

14. The composition according to claim 13, wherein the polyoxyethylene nonionic surfactant is selected from any one of polyoxyethylene 35 castor oil and polyoxyethylene 40 hydrogenated castor oil, or a mixture thereof.

15. The composition according to claim 14, wherein the polyoxyethylene nonionic surfactant is polyoxyethylene 35 castor oil.

16. The composition according to claim 1, wherein based on the total weight of the composition, the composition comprises 10%-50% of diethylene glycol monoethyl ether.

17. The composition according to claim 16, wherein based on the total weight of the composition, the composition comprises 15%-45% of diethylene glycol monoethyl ether.

18. The composition according to claim 17, wherein based on the total weight of the composition, the composition comprises 30%-45% of diethylene glycol monoethyl ether.

19. The composition according to claim 1, wherein based on the total weight of the composition, the composition comprises 5%-10% of dapsone and 0.1%-0.3% of adapalene.

20. The composition according to claim 19, wherein based on the total weight of the composition, the composition comprises 7.5% of dapsone and 0.1% of adapalene.

21. The composition according to claim 19, wherein based on the total weight of the composition, the composition comprises 6% of dapsone and 0.1% of adapalene.

22. The composition according to claim 19, wherein based on the total weight of the composition, the composition comprises 5% of dapsone and 0.1% of adapalene.

23. The composition according to claim 1, wherein the weight ratio of the polyoxyethylene nonionic surfactant to the carboxylic acid ester-based oily solvent is ≥1:1 or >1:1.

24. The composition according to claim 23, wherein the weight ratio of the polyoxyethylene nonionic surfactant to the carboxylic acid ester-based oily solvent is ≤3:1.

25. The composition according to claim 1, wherein the other pharmaceutically acceptable carriers comprise a crystallization inhibitor, and wherein the composition comprises 0.5%-4% of the crystallization inhibitor based on the total weight of the composition.

26. The composition according to claim 25, wherein the composition comprises 1%-3% of the crystallization inhibitor based on the total weight of the composition.

27. The composition according to claim 25, wherein the crystallization inhibitor is selected from any one of PVP, PVA, and HPC, or a combination thereof.

28. The composition according to claim 27, wherein the crystallization inhibitor is PVP.

29. The composition according to claim 28, wherein the PVP is PVP-K30.

30. The composition according to claim 1, wherein the pharmaceutically acceptable carrier further comprises a polymeric emulsifier, and based on the total weight of the composition, the composition comprises 0.05-6% of the polymeric emulsifier.

31. The composition according to claim 30, based on the total weight of the composition, wherein the composition comprises 0.05%-1% of the polymeric emulsifier.

32. The composition according to claim 31, based on the total weight of the composition, wherein the composition comprises 0.1%-0.5% of the polymeric emulsifier.

33. The composition according to claim 30, wherein the polymeric emulsifier is a carbomer copolymer.

34. The composition according to claim 33, wherein the carbomer copolymer is an acrylate /C10-30 alkyl acrylate crosspolymer.

35. The composition according to claim 1, wherein the pharmaceutically acceptable carrier further comprises a thickening agent, and based on the total weight of the composition, the composition comprises 0.1%-4% of the thickening agent.

36. The composition according to claim 35, wherein the thickening agent is a combination of SEPINEO P 600 and Carbomer 981.

37. The composition according to claim 36, based on the total weight of the composition, wherein the composition comprises 1%-3% of SEPINEO P 600 and 0.1%-1% of Carbomer 981.

38. The composition according to claim 1, wherein the oil droplet size of the composition is ≤300 nm.

39. The composition according to claim 1, wherein the composition is an emulgel.

40. The composition according to claim 1, wherein the composition is an emulsion.

41. The composition according to claim 1, wherein the pharmaceutically acceptable carrier further comprises any one selected from preservatives, antioxidants, chelating agents, and pH regulators, or a combination thereof.

42. The composition according to claim 41, wherein the preservative is methyl paraben.

43. The composition according to claim 41, wherein the antioxidant is butylated hydroxytoluene.

44. The composition according to claim 41, wherein the chelating agent is disodium edetate.

45. The composition according to claim 41, wherein the pH regulator is sodium hydroxide.

46. The composition according to claim 1, wherein the pH of the composition is in the range of 5.0-7.0, preferably 5.0-6.5, more preferably 5.0-6.0.

47. The method for preparing the composition according to any one of claims 1-46, wherein the preparation method includes the following steps:
1) preparing an oil phase: adding dapsone and adapalene into a mixed solution of a carboxylic acid ester-based oily solvent, a polyoxyethylene nonionic surfactant, and diethylene glycol monoethyl ether to form an oil phase;
2) preparing an aqueous phase: uniformly dispersing the other pharmaceutically acceptable carriers in water followed by mixing to form an aqueous phase; or directly use water as the aqueous phase
3) emulsification: adding the oil phase prepared in step 1) to the aqueous phase prepared in step 2), adjusting the pH to 5.0-6.0, mixing uniformly, and adding water to make up to 100%;
wherein, preferably, the other pharmaceutically acceptable carriers in step 2) are any one of a polymeric emulsifier, a thickening agent, and a crystallization inhibitor, or a mixture thereof;
wherein, preferably, the other pharmaceutically acceptable carriers in step 2) are a polymeric emulsifier, a thickening agent, and a crystallization inhibitor;
wherein, preferably, in step 2), the thickening agent is added after the polymeric emulsifier has been added;
wherein, preferably, the crystallization inhibitor is added prior to the addition of the polymeric emulsifier in step 2);
wherein, preferably, in step 1) and/or step 2), any one selected from a penetration enhancer, an antioxidant, a preservative, and a chelating agent, or a mixture thereof, is further added.

48. Use of a composition comprising adapalene and dapsone for the preparation of a medicament for treating skin diseases.

49. The use according to claim 48, wherein skin diseases are selected from the group consisting of acne vulgaris, rosacea, acne rosacea, chronic wounds, pressure sores, keratosis, sebaceous cysts, post-inflammatory hyperpigmentation, eczema, xerosis, pruritus, lichen planus, dermatitis, atopic dermatitis, prurigo nodularis, and miliaria.

50. The use according to claim 48 or 49, wherein the skin disease is acne vulgaris.

51. Use of a composition comprising adapalene and dapsone for the preparation of a topically administered pharmaceutical formulation.

52. The use according to claim 51, wherein the topically administered pharmaceutical formulation is selected from the group consisting of emulsions, emulgels, and creams.

53. A method for treating skin diseases, which comprises administering to a subject in need thereof a therapeutically effective amount of the composition comprising adapalene and dapsone according to any one of claims 1-46.

54. The method according to claim 53, wherein the skin diseases are selected from the group consisting of acne vulgaris, rosacea, acne rosacea, chronic wounds, pressure sores, keratosis, sebaceous cysts, post-inflammatory hyperpigmentation, eczema, xerosis, pruritus, lichen planus, dermatitis, atopic dermatitis, prurigo nodularis, and miliaria.

55. The method according to claim 53 or 54, wherein the skin disease is acne vulgaris.
